(19) **Europäisches Patentamt**

**European Patent Office**

**Office européen des brevets**

(11) **EP 1 108 417 B1**

(12) **EUROPEAN PATENT SPECIFICATION**

(45) Date of publication and mention
of the grant of the patent:
**05.07.2006 Bulletin 2006/27**

(51) Int Cl.:
*A61Q 5/10* *(2006.01)*  *A61K 8/34* *(2006.01)*

(21) Application number: **00126810.1**

(22) Date of filing: **07.12.2000**

(54) **Hair colorant composition**

Haarfärbezusammensetzung

Composition de teinture pour les cheveux

(84) Designated Contracting States:
**AT BE CH CY DE DK ES FI FR GB GR IE IT LI LU MC NL PT SE TR**

(30) Priority: **16.12.1999 EP 99125082**

(43) Date of publication of application:
**20.06.2001 Bulletin 2001/25**

(73) Proprietor: **DSM IP Assets B.V.**
**6411 TE Heerlen (NL)**

(72) Inventor: **Hickling, Maurice Raymond**
**Pertenhall,**
**Bedfordshire MK 44 2AT (GB)**

(74) Representative: **Schwander, Kuno**
**DSM Nutritional Products Ltd**
**Patent Department VMD**
**Bau 241 / 636**
**P.O. Box 3255**
**4002 Basel (CH)**

(56) References cited:
**EP-A- 0 706 788**    **WO-A-93/15708**
**DE-A- 4 234 744**    **DE-C- 19 755 491**

EP 1 108 417 B1

**Description**

The present invention relates to the use of phytantriol in a hair colorant composition to improve the wash fastness of dyed hair.

Phytantriol is chemically identified as 3,7,11,15-tetramethyl-1,2,3-hexadecanetriol and commercially available, e.g., from F. Hoffmann-La Roche AG, Basel, Switzerland.

US Patent 5,776,443 describes a hair care composition comprising 0.001-lwt% of phytantriol; 0.001-10 wt% of a silicone compound; and a carrier.

US Patent 5,834,013 describes a cosmetic or dermatological composition in the form of an aqueous and stable dispersion of cubic gel particles based on phytantriol and use thereof for hydrating the skin. Said composition essentially comprises: (a) from 0.1 to 15% by weight of phytantriol, and (b) from 0.1 to 3% by weight of a dispersing and stabilizing agent, e.g. Tween 20.

The above mentioned Patent Publications do not mention any effect of phytantriol concerning the wash fastness of hair dyes.

It has now been found that the addition of phytantriol to a hair colorant composition significantly improves the wash fastness of the hair dye, i.e., significantly improves the color durability of the dye on the hair upon washing.

Accordingly, the present invention relates to the use of phytantriol in a hair colorant composition to improve wash fastness of dyed hair.

More particularly, the present invention relates to the use of phytantriol in a hair colorant composition comprising from about 0.1 to about 5 % by weight of phytantriol, preferably from about 0.2 to about 1% by weight;

from about 0.1 to about 3% by weight of a dispersing agent, preferably from about 0.1 to about 1 % by weight; and

from about 0.1 to about 5 % by weight of a hair dye, preferably from about 0.1 to about 1 % by weight;

the remainder of the composition being essentially a carrier conventionally used in hair colorant compositions, suitably an aqueous carrier,

to improve the wash fastness of dyed hair.

The dispersing agent present in the compositions in accordance with the invention can be any dispersing agent (surfactant) conventionally used in hair dye compositions such as anionic, cationic, nonionic, amphoteric, zwitterionic (betain) surfactants and mixtures thereof.

Suitable dispersing agents are e.g. selected from:

(1) polyol alkyl or alkenyl ethers or esters,
(2) N-acylated amino acids and derivatives thereof and N-acylated peptides with an alkyl or alkenyl radical, and salts thereof,
(3) alkyl or alkenyl ether or ester sulfates, and derivatives and salts thereof,
(4) polyoxyethylenated alkyl or alkenyl fatty ethers or esters,
(5) polyoxyethylenated alkyl or alkenyl carboxylic acids and salts thereof,
(6) N-alkyl or N-alkenyl betaines,
(7) alkyltrimethylammonium or alkenyltrimethylammonium and salts thereof, and mixtures of the above dispersing agents.

In the compounds listed above, the alkyl and alkenyl radicals suitably have from 8 to 22 carbon atoms.

Preferred dispersing agents are polyol alkyl or alkenyl ethers or esters. Among these, there may in particular be mentioned sorbitan alkyl or alkenyl esters polyoxyethylenated with at least 20 units of ethylene oxide, such as sorbitan palmitate 20 EO or Polysorbate 40 marketed under the tradename Montanox 40 DF by the company Seppic, and sorbitan laurate 20 EO or Polysorbate 20 marketed under the tradename Tween 20 by the company ICI. Especially preferred is Tween 20.

Examples of suitable dyes include all conventional hair dyes, particularly permanent hair dyes, semipermanent hair dyes, and temporary hair dyes (or hair coloring rinses). Permanent (or oxidative) hair dyes comprise a component that requires an oxidizing agent for formation of a colour (said component being referred to also as primary intermediate, developer or precursor), usually a p-phenylenediamine, 2,5-diaminotoluene or 4-aminophenol, and a second component (secondary intermediate or coupler), e.g., a m-phenylenediamine, 3-aminophenol, 5-amino-2-methylphenol or 1-naphthol, as particularly disclosed e.g., in WO 00/07550. Semipermanent hair dyes comprise compouznds having a chromophore system common in dye chemistry such as nitro, azo, anthraquinone, triphenylmethane and azomethine, which may be used as disperse dyes, cationic (basic) dyes, anionic (acidic) dyes and metal complex dyes, see also Ullmann's Encyclopedia of Industrial Chemistry Vol A 12, p. 584-586 and references cited therein. Of particular interest are e.g. anthraquinone dyes, especially those containing a dialkylaminoalkylamino (DAAA) group. Specific examples include the following compounds available under the trademark ARIANOR® from Warner Jenkinson Ltd.: ARIANOR Straw Yellow, ARIANOR Mahagony, ARIANOR Steel Blue, ARIANOR Madder Red, ARIANOR Ebony and ARIANOR Sienna Brown.

Further examples of hair dyes for use in the present invention are those listed in the "Verordnung über kosmetische Mittel, Bundesgesetzblatt (Germany) I, 2410, 29. Aenderung vom 14.6.2000, Teil A, p. 846.

However any other hair dyes may be used. For example, semi-permanent (direct) hair dyes such as those commercially available under such tradenames as Irgalan, Cibalan, Vialon, Ortalan and Capracyl dyes. Natural direct dyestuffs, such as Henna, Camomile, Madder root, Sandalwood or Walnut, may also be used..

The formulation may for example be in the form of a liquid, a gel or a mousse. Specific examples of such formulations include a shampoo, a conditioner, a styling mousse, a styling spray, a styling gel, a conditioning styling gel, or a gel mousse. These formulations may, in addition to a hair colorant, contain agents conventionally used in such formulations, such as amphoteric surfactants, thickeners, conditioning agents, sequestrants (to remove heavy metals), preservatives, perfumes and deionized water.

The compositions are suitably prepared by dissolving an appropriate amount of phytantriol in an appropriate amount of dispersing agent, and adding the mixture to an essentially aqueous hair care formulation, such as a solution of the hair dye, a styling mousse, gel or spray, or a shampoo or conditioner. It is not required that the compositions comprise any preferred molecular orientation of the phytantriol.

When the hair dye is a permanent (or oxidative) hair dye the composition suitably comprises a kit of separately packed primary intermediate and secondary intermediate and, optionally, oxidising agent, at least one of the separate packs containing phytantriol and a dispersing agent in an amount defined earlier. Examples of oxidizing agents are hydrogen peroxide and organic peroxy compounds such as peracetic acid which may be used in an amount known to those skilled in the art, e.g., in an amount of from 0.001 to 0.05 moles per 100 g of the composition. Therefore, the invention also relates to the use of phytantriol in a hair colouring kit comprising

from about 0.1 to about 5 % by weight of phytantriol, preferably from about 0.2 to about 1% by weight;

from about 0.1 to about 3% by weight of a dispersing agent, preferably from about 0.1 to about 1 % by weight; and

from about 0.1 to about 5 % by weight of a hair dye, preferably from about 0.1 to about 1 % by weight;

wherein the hair dye consists of separately packed primary and secondary intermediate and wherein phytantriol and dispersing agent is present in at least one of the separate packs, to improve the wash fastness of dyed hair.

The composition may be applied to the hair by conventional techniques used in this art. e.g. with a brush, sponge, or other means of contact, such as pouring the composition directly onto the hair until saturated and/or manually massaging or working through the hair.

The advantages and characteristics of the present invention will be apparent from the examples presented below.

Example 1

Preparation of the hair colorant composition

0.2 g Phytantriol was dissolved in 0.8 g dispersing agent (Tween 20) and this mixture was added to 99.0 g of a 0.5% aqueous solution of the hair dye.

Example 2

The coloring composition was applied to approximately 10 cm long hair swatches for 10 minutes. The hair swatches were then washed with water and allowed to dry in the air. This cycle was repeated 5 times and 10 times.

Example 3

Color measurement

Hair colorant compositions containing 0.1 wt% or 0.2 wt% phytantriol have been prepared according to Example 1 and applied according to Example 2

Hair colors may be defined using the luminance (L), according to the colorimetric (L,a,b) coordinate system of the C.I.E (Comite International de l'Eclairage International Lighting Committee ).

In this color measurement method the color along three mutually perpendicular axes is measured photometrically and the color differences between the initial sample of hair before washing and the samples after 5 and 10 washes are compared.

The difference between the two samples in the CIELAB space is given by

$$\Delta E = \sqrt{[\Delta (\Delta a)^2 + (\Delta b)^2 + (\Delta L)^2]}$$

$\Delta E$     is the color difference
$\Delta a$     is the color difference along the a axis
$\Delta b$     is the color difference along the b axis
$\Delta L$     is the difference in luminance

The following Table shows the results:

| ARIANOR dye | % Phytantriol | $\Delta E$ after 10 washings |
| --- | --- | --- |
| A. Straw Yellow | 0 | 16.7 |
| | 0.1 | 15.5 |
| | 0.2 | 12.7 |
| A. Mahogony | 0 | 15.7 |
| | 0.1 | 15.4 |
| | 0.2 | 4.2 |
| A. Steel Blue | 0 | 27.0 |
| | 0.1 | 25.2 |
| | 0.2 | 14.3 |
| A. Madder Red | 0 | 35.8 |
| | 0.1 | 34.4 |
| | 0.2 | 20.7 |
| A. Ebony | 0 | 23.7 |
| | 0.1 | 23.4 |
| | 0.2 | 9.9 |
| A. Sienna Brown | 0 | 17.0 |
| | 0.1 | 16.9 |
| | 0.2 | 9.6 |

The above table clearly shows that the hair dye is more washfast when the hair dyeing treatment has been done using a hair colorant compositions containing phytantriol, especially in an amount of at least 0.2 wt%.

**Claims**

1.  Use of phytantriol in hair colorant composition comprising
    from about 0.1 to about 5 % by weight of phytantriol, preferably from about 0.2 to about 1% by weight;
    from about 0.1 to about 3% by weight of a dispersing agent, preferably from about 0.1 to about 1 % by weight; and
    from about 0.1 to about 5 %by weight of a hair dye, preferably from about 0.1 to about 1 % by weight,
    to improve the wash fastness of dyed hair.

2.  Use according to claim 1, wherein the dispersing agent is a polyol alkyl or alkenyl ether or ester.

3.  Use according to claim 2, wherein the dispersing agent is polysorbate 20.

4.  Use according to any one of claims 1-3 wherein the hair dye is a permanent dye.

**5.** Use according to any one of claims 1-3 wherein the hair dye is a semi-permanent dye.

**6.** Use according to claim 5 wherein the dye is an anthraquinone dye.

**7.** Use according to claim 6, wherein the anthraquinone dye contains a dialkylamino alkylamino group.

**8.** Use of phytantriol in a Hair colouring kit comprising a composition of
from about 0.1 to about 5 % by weight of phytantriol, preferably from about 0.2 to about 1% by weight;
from about 0.1 to about 3% by weight of a dispersing agent, preferably from about 0.1 to about 1 % by weight; and
from about 0.1 to about 5 % by weight of a hair dye, preferably from about 0.1 to about 1 % by weight;
wherein the hair dye consists of separately packed primary and secondary intermediate and wherein phytantriol
and dispersing agent is present in at least one of the separate packs,
to improve the wash fastness of dyed hair.


**Patentansprüche**

**1.** Verwendung von Phytantriol in einer Haarfärbezusammensetzung, umfassend
etwa 0,1 bis etwa 5 Gew.-% Phytantriol, bevorzugt etwa 0,2 bis etwa 1 Gew.-%;
etwa 0,1 bis etwa 3 Gew.-% eines Dispergiermittels, bevorzugt etwa 0,1 bis etwa 1 Gew.-%, und
etwa 0,1 bis etwa 5 Gew.-% eines Haarfarbstoffes, bevorzugt etwa 0,1 bis etwa 1 Gew-%; zur Verbesserung der
Waschechtheit des gefärbten Haares.

**2.** Verwendung nach Anspruch 1, wobei das Dispergiermittel ein Polyolalkyl- oder -Alkenylether oder -ester ist.

**3.** Verwendung nach Anspruch 2, wobei das Dispergiermittel Polysorbat 20 ist.

**4.** Verwendung nach einem der Ansprüche 1 bis 3, wobei der Haarfarbstoff ein Permanentfarbstoff ist.

**5.** Verwendung nach einem der Ansprüche 1 bis 3, wobei der Haarfarbstoff ein Halbpermanentfarbstoff ist.

**6.** Verwendung nach Anspruch 5, wobei der Farbstoff ein Anthrachinonfarbstoff ist.

**7.** Verwendung nach Anspruch 6, wobei der Anthrachinonfarbstoff eine Dialkylaminoalkylaminogruppe enthält.

**8.** Verwendung von Phytantriol in einem Haarfärbekit, umfassend eine Zusammensetzung aus:

etwa 0,1 bis etwa 5 Gew.-% Phytantriol, bevorzugt etwa 0,2 bis etwa 1 Gew.-%;
etwa 0,1 bis etwa 3 Gew.-% eines Dispergiermittels, bevorzugt etwa 0,1 bis etwa 1 Gew.-%, und
etwa 0,1 bis etwa 5 Gew.-% eines Haarfarbstoffes, bevorzugt etwa 0,1 bis etwa 1 Gew-%;
wobei der Haarfarbstoff aus separat verpacktem primärem und sekundärem Zwischenprodukt besteht, und
worin das Phytantriol und das Dispergiermittel zumindest in einer der separaten Verpackungen vorhanden ist,
zur Verbesserung der Waschechtheit des gefärbten Haares.


**Revendications**

**1.** Utilisation de phytantriol dans une composition de teinture pour les cheveux comprenant
d'environ 0,1 à environ 5 % en poids, de préférence d'environ 0,2 à environ 1 % en poids, de phytantriol ;
d'environ 0,1 à environ 3 % en poids, de préférence d'environ 0,1 à environ 1 % en poids, d'un agent dispersant ; et
d'environ 0,1 à environ 5 % en poids, de préférence d'environ 0,1 à environ 1 % en poids, d'une teinture pour les
cheveux ;
pour améliorer la tenue au lavage des cheveux teints.

**2.** Utilisation selon la revendication 1, dans laquelle l'agent dispersant est un polyol alkyl- ou alcényléther ou -ester.

**3.** Utilisation selon la revendication 2, dans laquelle l'agent dispersant est le Polysorbate 20.

**4.** Utilisation selon l'une quelconque des revendications 1 à 3 dans laquelle la teinture pour les cheveux est une teinture permanente.

**5.** Utilisation selon l'une quelconque des revendications 1 à 3, dans laquelle la teinture pour les cheveux est une teinture semi-permanente.

**6.** Utilisation selon la revendication 5, dans laquelle la teinture est une teinture anthraquinone.

**7.** Utilisation selon la revendication 6, dans laquelle la teinture anthraquinone contient un groupe dialkylaminoalkylamino.

**8.** Utilisation de phytantriol dans un ensemble de teinture comprenant une composition de
environ 0,1 à environ 5 % en poids, de préférence d'environ 0,2 à environ 1 % en poids, de phytantriol ;
environ 0,1 à environ 3 % en poids, de préférence d'environ 0,1 à environ 1 % en poids, d'un agent dispersant ; et
environ 0,1 à environ 5 % en poids, de préférence d'environ 0,1 à environ 1 % en poids, d'une teinture pour les cheveux ;
dans laquelle la teinture pour les cheveux est constituée d'un intermédiaire primaire et secondaire conditionnés séparément et dans lequel le phytantriol et l'agent dispersant sont présents dans au moins l'un des conditionnements séparés,
pour améliorer la tenue au lavage des cheveux teints.